# EUROPÄISCHE PATENTSCHRIFT

(11) **EP 0 966 486 B1**
(45) Veröffentlichungstag und Bekanntmachung des Hinweises auf die Patenterteilung: **13.04.2005**
(21) Anmeldenummer: 98966537.7
(22) Anmeldetag: 09.12.1998
(51) Int. Cl.: C08J 3/16, B01J 2/06, G01N 30/48

(54) **VERFAHREN ZUR HERSTELLUNG VON REGULÄREN PORÖSEN PERLCELLULOSEN**
METHOD FOR PRODUCING REGULAR POROUS CELLULOSE PEARLS
PROCEDE DE PRODUCTION DE CELLULOSE SOUS FORME DE PERLES POREUSES REGULIERES

(30) Priorität: 14.12.1997 DE 19755352; 14.12.1997 DE 19755353
(43) Veröffentlichungstag der Anmeldung: 29.12.1999
(73) Patentinhaber: THÜRINGISCHES INSTITUT FÜR TEXTIL- UND KUNSTSTOFF-FORSCHUNG e.V., 07407 Rudolstadt-Schwarza (DE)
(72) Erfinder: BEYER, Christine, D-07407 Rudolstadt (DE); MEISTER, Frank, D-07407 Rudolstadt (DE); MICHELS, Christoph, D-07407 Rudolstadt (DE); RIEDEL, Bernd, D-07318 Dorfkulm (DE); TAEGER, Eberhard, D-07407 Weissbach (DE)
(74) Vertreter: Brandenburg, Thomas, Dr.
(86) Internationale Anmeldenummer: PCT/DE1998/003657
(87) Internationale Veröffentlichungsnummer: WO 1999/031141

(56) Entgegenhaltungen:
- EP-A- 0 321 597
- EP-A- 0 425 477
- DE-A- 1 792 230
- DE-A- 19 522 181
- US-A- 5 245 024

## Beschreibung

Die Erfindung betrifft ein Verfahren zur Herstellung regulärer poröser Perlcellulosen mit einem Teilchengrößenbereich von 2 bis 1.000 µm. Die Erfindung betrifft ferner eine Perlcellulose mit bestimmten Eigenschaften sowie die Verwendung der nach dem erfindungsgemäßen Verfahren hergestellten Perlcellulosen.

Reguläre, poröse Perlcellulosen stellen im Vergleich zu anderen Trenn- und Trägermaterialien ein relativ preiswertes, stabiles Material mit vielseitig einstellbaren chemischen Eigenschaften dar. In zunehmenden Umfang gewinnen Celluloseformkörper als chromatographisches Material, als Träger für Enzyme, Zellen und andere Liganden, z. B. nach Aktivierung und Kopplung von Proteinen erheblich an Bedeutung.

Die bekannten Verfahren zur Herstellung derartiger Celluloseformkörper unterscheiden sich im wesentlichen in der Art des verwendeten Cellulosematerials, der verwendeten Lösungsmittel, der Art und Weise der Koagulation bzw. Regenerierung sowie der Zerteilungstechnologie.

So beschreiben die Schutzrechte JP 48-2173, JP 48-60753, JP 62-191033, CS 172640, US 2 543 928, DE 2005 408, u. a. die Verwendung von alkalischen Cellulosexanthogenatlösungen (Viskose), die entweder in ein saures Fällbad gesprüht oder nach Dipergieren in einem mit Wasser nicht mischbaren Lösungsmittel durch saure oder thermische Zersetzung regeneriert werden. Nachteilig bei dieser Vorgehensweise ist, daß von den bei der Regenerierung frei werdenden Schwefelverbindungen, den anfallenden Dünnsäuren sowie Salzlösungen bzw. durch die verwendeten organischen Lösungsmittel ein erhebliches Umweltgefährdungspotential ausgeht.

Andere Verfahren, beispielsweise nach DD 259 533, schlagen die Verwendung von Cellulosecarbamatlösungen vor. Dabei ist die Notwendigkeit einer kostenintensiven Nachbehandlung, bei der Harnstoff mit heißem Wasser entfernt und Restcarbamatgruppen mit Natronlauge zersetzt werden müssen, von besonderem Nachteil.

Eine weitere Gruppe von Schutzrechten geht von hochsubstituierten organolöslichen Celluloseestern aus, wobei bevorzugt Celluloseacetat mit durchschnittlichem Sustitutionsgrad (DS) zwischen 2 und 3 eingesetzt wird. Das Grundprinzip dieser über Celluloseacetatperlen als Zwischenprodukt verlaufenden Verfahren besteht darin, daß man Celluloseacetat bevorzugt in einem Halogenkohlenwasserstoff löst, die Polymerlösung dispergiert und durch Verdunsten des Lösungsmittels verfestigt. Nach dem Separieren der Cellulosacetatteilchen erfolgt in der Regel die Abspaltung der Acetatgruppen durch Behandlung mit Natronlauge, beispielsweise JP 53-7759. Da bei einem solchen Vorgehen nur Teilchen mit geringer Porosität erhalten werden, wurden eine Vielzahl von Verfahren vorgeschlagen, die das Erzeugen einer höheren Porosität der resultierenden Celluloseformkörper zum Ziel haben. Die Methode der Wahl ist dabei der Zusatz verschiedener Porenbildner zur Celluloseacetatlösung. Die Schutzrechte JP 56-24429, JP 24430, JP 62-267339, JP 63-68645 und US 4312980 schlagen die Verwendung von linearen Alkohlen vor. Motozato u. a. , J. Chromatogr. 298 (3), (1984) 499-507, bevorzugen hierfür Kohlenwasserstoffe, wie beispielsweise Hexan, Cyclohexan, Petrolether, Toluol u. ä.. Im Schutzrecht JP 63-68645 wird darüber hinaus die Verwendung von langkettigen Carbonsäuren bzw. Carbonsäureestern zu diesem Zweck vorgeschlagen. Nachteilig bei all diesen Modifizierungen bleibt die Notwendigkeit der Verwendung von toxischen Halogenkohlenwasserstoffen als Lösungsmittel.

Das Verfahren der Schutzrechte SU 931 727 und SU 1031 966, die eine Herstellung von Celluloseperlen ausgehend von Celluloseacetat mit einem DS von 2 aus einer Ethylacetat-n-Butanol-Mischung zum Gegenstand haben, erlaubt keine Einstellung von Porositäten < 75 %. Die vorgeschlagene Verwendung von Ölsäure erfordert zusätzliche Waschprozesse unter Einsatz flüchtiger organischer Lösungsmittel.

Ein Vorgehen, wie im Schutzrecht DD 295 861 aufgezeigt, zur Herstellung von perlförmigen Cellulosepartikeln unter Verwendung von Cellulosesilylethern benutzt ebenfalls flüchtige Kohlenwasserstoffe bzw. toxische Halogenkohlenwasserstoffe als Lösungsmittel. Bei der sauren bzw. alkalischen Regenerierung verbleiben merkliche Anteile an Silylseitengruppen, die eine Anwendung für chromatographische bzw. medizinische Zwecke erheblich einschränken.

Für die direkte Auflösung der Cellulose wurden bisher schlecht handhabbare Lösungsmittel vorgeschlagen. So beschreiben die Schutzrecht DE 1792 230, FR 1575419, US 3 597 350 die Verwendung von Cuoxam u. ä..

Das Schutzrecht JP 80-44312 sowie Kuga, J. Chromatogr. 195, (1980), 221-230 schlagen ein Arbeiten in CaSCN-Schmelzen vor.

Weiterhin werden in JP 82 - 159802 Dimethylsulfoxid-Paraformaldehyd-Mischungen als Lösungsmittel beschrieben. Insbesondere die Mehrkomponentenlösungsmittel bereiten erhebliche Probleme beim Einbringen von höhermolekularen Cellulosen in Mengen über 5 %. Weiterhin lassen sich diese Lösungsmittelgemische nur sehr eingeschränkt recyclisieren.

In Bezug auf die Zerteilung der Polymerlösung nach Austritt aus einer Düse werden im wesentlichen 3 Technologien beschrieben. So lehren beispielsweise die Schutzrechte US 5 047 180 und US 5 328 603 die Herstellung von sphärischen Formkörpern durch Zerstäuben (Atomisieren) einer Polymerlösung. Im zuletzt genannten Schutzrecht wird darüber hinaus das Mehrkomponentenlösungsmittel Dimethylacetamid / LiCl als Celluloselösungsmittel verwendet. Ein solches System erfordert für die Herstellung regulärer Partikel Salzzusätze über 10 % . Das Schutzrecht EP 0268 866 realisiert die Zerteilung in Polymertropfen durch die Überlagerung der Längsbewegung der aus der Düse austretenden Polymerlösung durch eine rotierende Vibrationsbewegung. Im Schutzrecht DE 44 24 998 schließlich werden sphärische Partikel durch das Zerteilen einer aus der Düse austretenden Polymerlösung mittels extrem dünner, rotierender Schneidmesser hergestellt. Allen Verfahrensvarianten ist gleich, daß sich unmittelbar an das Zerteilen der Polymerlösung ein irreversibler Koalgulationssschritt anschließt. Damit ist es notwendig, daß die Polymerpartikel beim Passieren einer mehr oder weniger kurzen Fallstrecke die reguläre Form annehmen. Dies führt zu Problemen bei der Ausbildung einer idealen Kugelgestalt durch vorzeitiges Aushärten, Deformationen beim Aufprall auf den umschließenden Auffangzylinder und u. U. Verklebungen auf den Schneidmessern, so daß mehr oder wenig starke Abweichungen in Kauf genommen werden müssen.

Der Erfindung liegt die Aufgabe zu Grunde, ein Verfahren zur Herstellung von regulären, porösen Celluloseperlen zu schaffen, das technisch einfach und wirtschaftlich ist und die Erzeugung von Perlkörpern mit definiertem Teilchendurchmesser bei enger Teilchengrößenverteilung im Gesamtbereich von 2 bis 1.000 µm und bei hoher Variationsbreite der einstellbaren Porositäten gestattet. Insbesondere soll das Verfahren Celluloseperlen mit Teilchengrößen in dem Teilbereich von 2 bis 50 µm oder dem Teilbereich von 40 bis 1.000 µm erlauben. Vorzugsweise sollen dabei wenig oder nicht toxische, salzfreie Lösungsmittel, insbesondere Einkomponentenlösungsmittel zum Einsatz kommen. Darüber hinaus soll ein Verfahren geschaffen werden, bei dem die aufgezeigten Nachteile der bekannten Verfahren vermieden werden. Schließlich soll eine neue Perlcellulose mit neuen Verwendungen geschaffen werden. Weitere Vorteile ergeben sich aus der folgenden Beschreibung.

Diese Aufgabe wird bei dem eingangs genannten Verfahren erfindungsgemäß dadurch gelöst, daß man
a) eine Cellulose mit einem Polymerisationsgrad in dem Bereich von 150 bis 2.000 in einem Lösungsmittel zu einer 0,5 bis 25 Masse-prozentigen Lösung auflöst,
b) die Celluloselösung fein zerteilt und in einem nicht mit ihr mischbaren, eine Viskosität in dem Bereich von 10 bis 80.000 mPa.s aufweisenden Dispersionsmittel dispergiert,
c) die dispersen Lösungsteilchen
   1) nach Abkühlung der Dispersion unter die Schmelztemperatur der Celluloselösung und Abtrennung der erstarrten Celluloselösungsteilchen von dem Dispersionsmittel oder
   2) direkt in der Dispersion
   durch Ausfällen mit einem mit dem Lösungsmittel mischbaren flüssigen Fällungsmittel zu regulären Perlteilchen verfestigt, und
d) die Perlteilchen von dem flüssigen Gemisch aus Lösungmittel, Fällungsmittel und ggf. Dispersionsmittel abtrennt.

Bei einer bevorzugten Ausführungsform des erfindungsgemäßen Verfahrens wird zur Herstellung von Perlcellulosen mit einem Teilchengrößenbereich von 2 bis 50 µm die Lösung mit 0,5 bis 15 Masseprozent Cellulose direkt in einem flüssigen, inerten Medium dispergiert und die Dispersion durch die Verfahrensweise der Stufen c1) und d) weiterverarbeitet. Es wurde gefunden, daß die Trennung von Formbildung und Verfestigung durch die Umwandlung der Cellulosedispersion nach Temperaturerniedrigung in eine Suspension zu wesentlichen Vereinfachungen im Herstellungsprozeß führt.

Bei einer anderen bevorzugten Ausführungsform des erfindungsgemäßen Verfahrens zur Herstellung von Perlcellulosen mit einem Teilchengrößenbereich von 50 bis 1.000 µm wird die Celluloselösung in der Stufe b) durch Druck zu wenigstens einem Strahl mit einem Durchmesser in dem Bereich von 40 bis 1.000 µm verformt, der Lösungsstrahl mit Hilfe rotierender Schneidstrahlen in definierte Segmente zerteilt und werden diese Lösungsteilchen in dem Dispersionsmittel aufgefangen und in Bewegung gehalten. Die Trennung der Prozeßschritte Zerteilen, Formbildung und Verfestigung führt auf einfache Weise zu hochregulärer Perlcellulose enger Teilchengrößenverteilung und zu variabel einstellbaren Porenvolumina und erlaubt eine wesentliche Vereinfachung des Herstellungsprozesses.

Weitere Ausführungsformen des erfindungsgemäßen Verfahrens sind durch die Unteransprüche 4 bis 28 definiert. So kann man das nicht mit Wasser mischbare, inerte Zerteil- bzw. Dispersionsmedium nach Phasentrennung der Suspension direkt, d. h. ohne zusätzliche Reinigungsstufen, wie z. B. Extrahieren usw., besonders vorteilhaft in den Verfahrenskreislauf zurückführen und ggf. nach Zusatz von Dispergierhilfsmitteln erneut für den Formbildungsprozeß nutzen.

Die nach der Filtration bzw. Zentrifugation abgetrennten, erstarrten Polymertröpfchen lassen sich vorteilhaft unter Erhalt der in der Formbildungsstufe erzeugten Eigenschaften in einem Fällbad verfestigen. Das abgetrennte Lösungsmittel/Fällungsmittelgemisch kann beispielsweise durch Einsatz thermischer Energie oder durch Membranen aufgetrennt werden, so daß auch das Einkomponentenlösungsmittel vorteilhaft in kurzer Flotte zirkulieren kann.

Die Konzentration der Celluloselösung ist in weiten Grenzen in Abhängigkeit von der angestrebten Teilchengröße bzw. den Porenvolumina einstellbar, wobei zur Herstellung der erfindungsgemäßen regulären, porösen Perlcellulosen mit Teilchendurchmessern von 2 bis 50 µm bzw. 50 bis 1.000 µm Lösungen mit einer Cellulosekonzentration von 0,5 bis 15, vorzugsweise 1 bis 12, besonders bevorzugt von 2 bis 7 Masseprozent geeignet sind.

Die bei der Perlcelluloseherstellung in dem Größenbereich von 50 bis 1.000 µm benutzte Technik ohne rotierende Schneidmesser minimiert die Gefahr einer Verklebung des Schneidwerkzeugs mit Polymertröpfchen und trägt zusätzlich zur hohen Partikeleinheitlichkeit bei. Zur Teilchenvergleichmäßigung und Bildung einheitlicher Teilchengrößen ist es vorteilhaft, während und/oder nach dem Eintrag der Cellulosetröpfchen in das Dispergiermedium die Zerteilung mit einem üblichen Rührer solange aufrechtzuerhalten, bis durch Fällung bzw. Temperaturabsenkung eine stabile Suspension entstanden ist.

Bei der Erzeugung von Teilchen in dem Bereich von 2 bis 50 µm werden Viskositätsgradienten zwischen der Celluloselösung und dem Dispersionsmedium ausgenutzt. Zur Teilchenvergleichmäßigung und Bildung kleiner Teilchengrößen werden während und/oder nach dem Eintrag der Celluloselösung in das Dispergiermedium schnell rotierende Dispergiergeräte, beispielsweise vom Typ Ultra-Turrax im Drehzahlbereich von 1.000 bis 10.000 min⁻¹ mit intensiv wirkenden Dispergierwerkzeugen, beispielsweise mit Schneid-Misch-Kopf oder Dispergierstäben eingesetzt. Anschließend läßt sich die Zerteilung/Dispersion mit einem üblichen Rührer solange aufrechterhalten, bis durch Temperaturabsenkung eine stabile Suspension entstanden ist.

Die ggf. erstarrten Polymertröpfchen werden durch Filtration oder Zentrifugieren abgetrennt und anschließend einem Fällbad zugeführt, in welchem sie verfestigt werden. Nach der Abtrennung der erhaltenen regulären, porösen Celluloseperlen vom Fällbad durch Filtration oder Zentrifugieren wird eine Wäsche/Reinigung mit Wasser oder niederen Alkoholen im Temperaturbereich von 3 bis 90°C durchgeführt.

Zweckmässigerweise setzt dem inerten Zerteil- bzw. Dispersionsmedium geeignete Emulgatoren, wie nichtionische Tenside aus der Gruppe der Polyoxyethylenalkylether, Polyoxyethylenarylalkylether oder Polyoxyethylensorbitanalkylether zu.

Die Perlcellulosen können anschließend aktiviert und ggf. über Spacer mit unterschiedlichen Liganden gekoppelt werden. Bei Bedarf werden sie getrocknet. Die erfindungsgemäßen Perlcellulosen sind durch einen Teilchengrößenbereich von 50 bis 1.000 µm, ein Porenvolumen von 5 bis 95 % und eine Ausschlußgrenze ≤ 5 x 10⁶ Dalton oder aber durch einen Teilchengrößenbereich von 2 bis 50 µm, ein Porenvolumen < 50 % und eine Ausschlußgrenze ≤ 5 x 10⁴ Dalton gekennzeichnet. Durch das erfindungsgemäße Verfahren bzw. die betreffende Ausführungsform des Verfahrens sind diese Eigenschaften in den genannten Bereichen jeweils eng einstellbar. Das Porenvolumen beschreibt den Anteil einer Cellulosekugel, der durch mehr oder weniger große Hohlräume gekennzeichnet ist. Das Porenvolumen läßt sich durch Elektronenmikroskopie verschiedener Schnitte durch die Cellulosekugel, durch Quecksilberporosimetrie oder bei bekannter Abhängigkeit mit dem Wasserrückhaltevermögen - CRC-Wert (DIN 53814) - bestimmen.

Die erfindungsgemäßen Perlcellulosen, insbesondere die mit einem Teilchengrößenbereich von 2 bis 50 µm, einem Porenvolumen von weniger als 50 % und einer Ausschlußgrenze von ≤ 5 x 10⁴ Dalton können vorteilhaft zum Beispiel als Trennmittel und Trägermittel für chromatographische und diagnostische Zwecke, z. B. für Diagnostika und Biokatalysatoren, als selektives oder spezifisches Adsorbens bei der Blutdetoxikation und als Zellkulturträger in der Biotechnologie, Biomedizin und Medizin eingesetzt werden. Besonders ist die Perlcellulose als Matrix für die Gelfiltrationschromatographie (GFC) geeignet, bei der aufgrund des Porendurchmessers bzw. der Ausschlußgrenze Moleküle, hauptsächlich Makromoleküle getrennt werden.

Die Ausschlußgrenze charakterisiert jene Grenze der Größe eines Hohlraumes (Pore), bis zu der ein Molekül z. T. auch nur partiell in diesen Hohlraum einzudringen vermag. Sie stellt also die größtmögliche Dimension eines Moleküles dar, für die eine chromatographische Trennung noch möglich ist. Die Ausschlußgrenze wird mit Hilfe der Permeationsmessung bekannter Substanzen mit definierter Molekülgröße bestimmt. Im vorliegenden Fall werden Ausschlußgrenzen an Hand der Permeation von hochmolekularem Dextranblau ermittelt (vgl. J. Baldrian u. a.: "Small-angle scattering from macroporous polymers: styrene divinylbenzene copolymers, cellulose in bead form" in Coll. Chechoslov. Chem. Commun. 41 (1976) 12, S. 3555-3562). Teilchengröße, Porenvolumen und Ausschlußgrenze werden von Herstellern und Anwendern immer in ihrer Gesamtheit zur Produktcharakterisierung und Vergleichbarkeit der verschiedenen Erzeugnisse genutzt.

Der hier gebrauchte Terminus "regulär" bedeutet gleichmäßig im Sinne der Einheitlichkeit der geometrischen Form. Ideal geformte Kugeln gestatten eine optimale Packungsdichte (hexagonal dichteste Kugelpackung). Bei chromatographischen Trennprozessen können mit "regulären" Perlen gute Fließbedingungen für die zu trennende Phase und eine gute mechanische Stabilität der Packung erreicht werden. Im Ergebnis werden dann engverteilte Trennkurven bestimmt. Die Verwendung irregulärer Partikel (ungleichmäßige geometrische Form) hat große Totvolumina (auf der Säule länger verbleibende Phase, Verschleppen der verschiedenen Fraktionen) und eine geringe mechanische Stabilität zur Folge. Die Trennkurven haben eine breite Verteilung und zeigen ein sogenanntes "Tailing".

Die Erfindung wird durch die folgenden Beispiele näher erläutert.

### Beispiel 1

In einem Planschliffgefäß werden 16,5 g naß aufgeschlagene Cellulose mit einem Cuoxam-DP von 482 und einem Wassergehalt von 60 % sowie 1163 g einer 50 %igen wäßrigen N-Methylmorpholin-N-oxid-Lösung (NMMNO-Lösung) 60 min. intensiv bei 85°C gerührt. Anschließend werden bei konstanter Temperatur unter weiterem Rühren 80 ml Wasser im Vakuum entzogen, wobei eine Celluloselösung mit 6 Ma-% Cellulose entsteht. Die Polymerlösung wird bei 75°C in 200g Paraffinöl (Viskosität > 110 mPa.s), dem 2,5 g Tween® 80 (Polyoxyethylenlaurylether, Produkt der Firma ICI) zugefügt wurde, mit einem Ultra-Turrax mit einem Schneid-Mischkopf-Werkzeug bei 9.000 min⁻¹ dispergiert. Zur Aufrechterhaltung der Dispersion wird mit einem Rührwerk bei 250 min⁻¹ unter Abkühlen auf 35°C nachgerührt, wobei die Polymertröpfchen erstarren. Die so erhaltene Suspension wird durch Zentrifugieren getrennt, und die erstarrten Polymertröpfchen werden bei Raumtemperatur in ein wäßriges Fällbad, das 70 % Isopropanol enthält, überführt. Das abgetrennte Dipersionsmedium wird erneut zum Dispergieren genutzt. Die Celluloseperlen haben einen mittleren Teilchendurchmesser von 25 µm und ein Porenvolumen von annähernd 40 %.

### Beispiel 2

In einem Planschliffgefäß werden 7,5 g naß aufgeschlagene Cellulose mit einem Cuoxam-DP von 1.634 und 168,2 g einer 50 %igen wäßrigen NMMNO-Lösung gegeben. Zur Lösungsherstellung werden der Mischung bei 85°C unter Vakuum 76 ml Wasser entzogen, wobei eine 3 %ige Celluloselösung entsteht. Zur Herstellung der Perlcellulose wird analog Beispiel 1 verfahren.
Die so erhaltene Perlcellulose hat einen mittleren Teilchendurchmesser von 15 µm, ein Porenvolumen von 47 % und eine Ausschlußgrenze von 2 x 10³ Dalton.

### Beispiel 3

7,5 g naß aufgeschlagene Cellulose analog Beispiel 1 werden in einem Planschliffgefäß in 168,2 g 50 %iges, wäßriges NMMNO, in dem zuvor 1,0 g ZnO unter intensivem Rühren verteilt wurde, eingetragen und bei 85°C 60 min intensiv gerührt. Unter Vakuum werden 76 ml Wasser entzogen, so daß eine nahezu 3 %ige Celluloselösung entsteht. Die Polymerlösung wird bei 75°C in 200 g Siliconöl (Viskosität 53 mPa.s), dem 3 g Brij® 35 zugesetzt wurden, mit einem Ultra-Turrax bei 10.000 min⁻¹ dispergiert. Nach 15 min. wird der Ultra-Turrax abgeschaltet und die entstandene Dispersion unter Rühren mit 250 min⁻¹ auf 25°C abgekühlt. Nach Abzentrifugieren der erstarrten Polymertröpfchen werden nach Fällen im wäßrigen Bad Celluloseperlen mit einem mittleren Teilchendurchmesser von 10 µm und 8 % Porenvolumen erhalten. Die Oberfläche der Formkörper zeichnet sich durch eine geringe Porosität aus.

### Beispiel 4

11 g einer mikrokristallinen Cellulose mit einem Cuoxam-DP von 150 und einem Restfeuchtegehalt von 12 % werden in 90 g NMMNO-Monohydrat eingetragen und unter Rühren bei 90°C homogen gelöst. Die Polymerlösung wird in 200 g Paraffinöl mit einer Viskosität bei 20°C von > 110 mPa.s, das 2,5 g Tween® 85 (Polyoxyethylensorbitantrioleat) enthält, bei 80°C mit einem Ultra-Turrax und Dispersionswerkzeug bei 9.500 min⁻¹ dispergiert. Nach 25 min. wird der Ultra-Turrax abgeschaltet und die entstandene Dispersion unter Abkühlen bei 250 min⁻¹ weiter gerührt. Nach 35 minütigem, langsamen Rühren der Dispersion bei 35°C zentrifugiert man die erstarrten Polymertröpfchen ab und trägt sie zur Verfestigung in ein wäßriges Fällbad ein, dem 70 % Isopropanol zugesetzt wurden. Die erhaltene Perlcellulose hat einen mittleren Teilchendurchmesser von 5 µm und ein Porenvolumen von 10 %.

### Beispiel 5

4 g trocken gemahlener Zellstoff mit einem Cuoxam-DP von 1.634 werden bei Raumtemperatur in 196 g Trifluoressigsäure (98 %ig, Kp. = 72°C) langsam unter Rühren eingetragen. Unter langsamem Rühren bei 25°C wird der Zellstoff in einem Planschliffgefäß mit Rückflußkühler aufgelöst. Zur Vervollständigung der Auflösung wird nach 2 h auf 50°C erwärmt und weitere 30 Minuten gerührt. Dann wird wieder auf Raumtemperatur abgekühlt. Die Celluloselösung wird in 200 g Paraffinöl mit einer Viskosität bei 20°C von 25 bis 50 mPa.s, das 2,5 g Tween® 85 enthält, bei 25°C mit einem Ultra-Turrax und Dispergierwerkzeug bei 9.500 min⁻¹ dispergiert. Nach 25 min. wird der Ultra-Turrax abgeschaltet und die entstandene Dispersion unter Abkühlen auf ∼20°C bei 250 min⁻¹ weiter gerührt. Die erstarrten Polymertröpfchen werden bei dieser Temperatur abzentrifugiert und zur Fällung in ein alkoholisches Bad, das Isopropanol und tert.Butanol in einem Verhältnis 50/50 (V/V) enthält, eingetragen. Die erhaltenen Celluloseperlen besitzen einen mittleren Teilchendurchmesser von 10 µm und ein Porenvolumen von 10 %.

### Beispiel 6

In einem Planschliffgefäß werden 12,5 g naß aufgeschlagene Cellulose mit einem Cuoxam-DP von 1,634 und einem Wassergehalt von 60 % zusammen mit 252 g einer 50 %igen wäßrigen N-Methylmorpholin-N-oxid-Lösung bei 85°C intensiv gerührt. Anschließend destilliert man 85 g Wasser unter Vakuum und Rühren ab und setzt 50 g wasserfreien DMSO zu. Zur Vervollständigung der Auflösung werden weitere 30 g Wasser abdestilliert, so daß eine 2,5 %ige Celluloselösung entsteht.
Die Polymerlösung wird bei 70°C durch ein Düsenloch mit einem Durchmesser von 50 µm gepreßt und der abgespritzte Lösungsstrang durch einen unter 100 bar stehenden, rotierenden Flüssigkeitsstrahl aus Paraffinöl zu regulären, zylindrischen Segmenten mit einer Höhe von ca. 45 µm zerteilt. Die in Spritzrichtung herabfallenden Lösungspartikel werden in einem zylindrischen Gefäß aufgefangen, das ein Dispersionsmedium aus 300 g Paraffinöl enthält, dem 1 g Brij® 35 (Polyoxyethylenlaurylether, nicht-ionischer Emulgator der Firma ICI) hinzugefügt wurde. Die zylindrischen Segmente werden bei einer Temperatur von 75°C unter langsamen Rühren (200 min⁻¹) zu Lösungströpfchen geformt, die nach Absenken der Temperatur auf 10°C zu regulären, festen Partikeln erstarren. Die festen Teilchen werden vom Dipersionsmedium abfiltriert und anschließend in einem wäßrigen Fällbad, das 50 Ma-% DMSO enthält, verfestigt. Die erhaltene Perlcellulose besitzt einen mittleren Teilchendurchmesser von 55 µm ± 10 %, und ein Porenvolumen von ca. 65 %.

### Beispiel 7

25 g Cellulose mit einem DP von 482 und 60 % Restfeuchte werden mit 163 g 50%iger NMMNO-Lösung und 5g Polyethylenglykol mit einem Molekulargewicht von 35.000 intensiv bei 85°C gemischt. Unter Vakuum und Rühren wird dem Gemisch bei konstanter Temperatur 80 ml Wasser entzogen, so daß eine feindisperse Emulsion aus Celluloselösung und PEG entsteht. Die Emulsion wird ohne weitere Verweilzeit durch ein Düsenloch mit einem Durchmesser von 50 gepreßt und der abgespritzte Strang in gleicher Weise wie im Beispiel 6 in Segmente zerteilt. Diese werden in einem Polyalkylsiloxan mit einer Viskosität von 50 Pa.s, daß 2,5 g Emulgator (Brij® 35) enthält, aufgefangen. In der unter langsamen Rühren auf 50°C abgekühlten Dispersion werden die geformten Polymertröpfchen durch Zusetzen von 1.500 g entsalztem Wasser gefällt. Das in den Perlcellulosen verbliebene PEG wird mittels Heißwasserextraktion vollständig entfernt, so daß reguläre Partikel mit 60 µm Durchmesser und einem Porenvolumen von 83 % erhalten werden.

### Beispiel 8

15 g Cellulose analog Beispiel 6 werden mit 163 g 50%iger NMMNO-Lösung bei 80°C gemischt und im Vakuum unter Entzug von 80 ml Wasser aufgelöst. Zur Celluloselösung gibt man anschließend bei gleicher Temperatur 50 g ε-Caprolactam und rührt solange weiter bis eine homogene Masse entstanden ist. Die Polymerlösung wird bei 75°C durch ein Düsenloch mit einem Durchmesser von 100 µm gepreßt und analog Beispiel 6 zu zylinderförmigen Polymersegmenten zerteilt. Die nach langsamen Rühren geformten Polymertröpfchen läßt man durch Absenken der Temperatur der Dispersion auf 35°C erstarren, filtriert den Feststoff ab und fällt in ein Bad, das 50 % Caprolactam und 50 % Isopropanol enthält. Die Celluloseperlen besitzen einen Teilchendurchmesser von 150 µm und ein Porenvolumen von 87 %.

### Beispiel 9

Es wird analog Beispiel 6 gearbeitet, jedoch eine Cellulose mit einem DP von 532 eingesetzt. Die erhaltenen Celluloseperlen haben bei gleichem Teilchendurchmesser ein Porenvolumen von 70 %.

### Beispiel 10

Es wird analog zu Beispiel 8 gearbeitet, jedoch anstelle von Caprolactam 2 g hydrolysierte Markerbsenstärke mit einem Molekulargewicht von 240.000 in die Polymerlösung eingearbeitet, indem die Markerbsenstärke in der wäßrigen NMMNO-Lösung feinverteilt vorgequollen und die naß aufgeschlagene Cellulose anschließend eingetragen wird. Die erhaltenen Celluloseperlen haben einen Teilchendurchmesser im Mittel von 175 µm und ein Porenvolumen von 80 %, wobei größtenteils Meso- und Makroporen gebildet werden.

### Beispiel 11

4 g trocken gemahlener Linters-Zellstoff mit einem DP von 1.634 wird bei Raumtemperatur in 96 g Trifluoressigsäure (98 %ig, Kₚ = 72°C) in einem Planschliffgefäß mit Rührer und Rückflußkühler eingetragen und bei 25°C 2 h gerührt. Zur Vervollständigung der Auflösung wird auf 50°C erwärmt und weitere 30 min. gerührt. Die Lösung wird nach Abkühlen auf Raumtemperatur analog Beispiel 6 zerteilt und unter Abkühlen auf -20°C bei 250 min⁻¹ weiter gerührt. Die erstarrten Polymertröpfchen werden bei dieser Temperartur abzentrifugiert und zur Fällung in ein alkoholisches Bad, das Isopropanol und tert.-Butanol in einem Verhältnis 50/50 (V/V) enthält, eingetragen. Die Perlcellulose hat einen mittleren Teilchendurchmesser von 60 µm und ein Porenvolumen von 10%.

## Patentansprüche

1. Verfahren zur Herstellung regulärer, poröser Perlcellulose mit einem Teilchengrößenbereich von 2 bis 1.000 µm, **dadurch gekennzeichnet, daß** man
a) eine Cellulose mit einem Polymerisationsgrad in dem Bereich von 150 bis 2.000 in einem Lösungsmittel zu einer 0,5 bis 25 Masse-%igen Lösung auflöst,
b) die Celluloselösung fein zerteilt und in einem nicht mit ihr mischbaren, eine Viskosität in dem Bereich von 10 bis 80.000 mPa.s aufweisenden Dispersionsmittel dispergiert,
c) die dispersen Lösungsteilchen
1) nach Abkühlung der Dispersion unter die Schmelztemperatur der Celluloselösung und Abtrennung der erstarrten Celluloselösungsteilchen von dem Dispersionsmittel oder
2) direkt in der Dispersion
durch Ausfällen mit einem mit dem Lösungsmittel mischbaren flüssigen Fällungsmittel zu regulären Perlteilchen verfestigt, und
d) die Perlteilchen von dem flüssigen Gemisch aus Lösungmittel, Fällungsmittel und ggf. Dispersionsmittel abtrennt.

2. Verfahren nach Anspruch 1, **dadurch gekennzeichnet, daß** man zur Herstellung von Percellulosen mit einem Teilchengrößenbereich von 2 bis 50 µm die Lösung mit 0,5 bis 15 Masse-% Cellulose direkt in einem flüssigen, inerten Medium dispergiert und die Dispersion nach den Stufen c1) und d) weiterverarbeitet.

3. Verfahren nach Anspruch 1, **dadurch gekennzeichnet, daß** man zur Herstellung von Perlcellulosen mit einem Teilchengrößenbereich von 50 bis 1.000 µm die Celluloselösung in der Stufe b)
durch Druck zu wenigstens einem Strahl mit einem Durchmesser in dem Bereich von 40 bis 1.000 µm verformt,
den Lösungsstrahl mit Hilfe rotierender Schneidstrahlen in definierte Segmente zerteilt, und
die Lösungsteilchen in dem Dispersionsmittel auffängt und in Bewegung hält.

4. Verfahren nach einem der Ansprüche 1 bis 3, **dadurch gekennzeichnet, daß** das Lösungsmittel mit Wasser mischbar ist, das Dispersionsmittel wasserfrei und nicht mit Wasser mischbar ist und das Fällungsmittel wenigstens zum Teil aus Wasser besteht.

5. Verfahren nach einem der Ansprüche 1 bis 4, **dadurch gekennzeichnet, daß** das Lösungsmittel salzfrei ist und/oder das Fällungsmittel eine wässrige Salzlösung ist.

6. Verfahren nach einem der Ansprüche 1 bis 5, **dadurch gekennzeichnet, daß** man in Stufe a) 5 bis 200 Masse-% , bezogen auf die Cellulose, wenigstens eines inerten Feststoffs zusetzt.

7. Verfahren nach einem der Ansprüche 1 bis 6, **dadurch gekennzeichnet, daß** man in der Stufe a) ein pulverförmiges Material mit Teilchendurchmessern in dem Bereich von 50 bis 3.000 nm zusetzt.

8. Verfahren nach Anspruch 6 oder 7, **dadurch gekennzeichnet, daß** man den Feststoff unter Polysacchariden, wie Stärke, Xanthan und Galaktomannanen, und anorganischen Verbindungen, wie Zinkoxid, mit Teilchendurchmessern von < 40 nm auswählt.

9. Verfahren nach einem der Ansprüche 3 bis 8, **dadurch gekennzeichnet, daß** man in de Stufe a) wenigstens ein inertes Verdünnungsmittel aus der aus Dimethylsulfoxid, Laktamen, Polyethylenglykol und Polypropylenglykol bestehenden Gruppe zusetzt.

10. Verfahren nach einem der Ansprüche 1 bis 9, **dadurch gekennzeichnet, daß** man das Dispersionsmittel aus der aus Polyalkylsiloxanen, Paraffinen und Polypropylenglykolen bestehenden Gruppe auswählt.

11. Verfahren nach einem der Ansprüche 2 bis 8, **dadurch gekennzeichnet, daß** man die Stufe b) unter der Wirkung eines Scherfeldes eines Dispergierwerkzeugs durchführt, das mit einer Drehzahl n in dem Bereich von 10³ bis 10⁴ min⁻¹ rotiert.

12. Verfahren nach Anspruch 3, **dadurch gekennzeichnet, daß** man die Celluloselösung durch wenigstens eine Düse extrudiert und die Schneidstrahlen durch eine komprimierte inerte Flüssigkeit erzeugt.

13. Verfahren nach einem der Ansprüche 1 bis 12, **dadurch gekennzeichnet, daß** man in der Stufe b) 1 bis 30 Masse-%, bezogen auf eingesetzte Cellulose, wenigstens eines Emulgators zusetzt.

14. Verfahren nach Anspruch 13, **dadurch gekennzeichnet, daß** man den Emulgator unter nichtionischen Tensiden der aus Polyoxyethylenalkylether, Polyoxyethylenarylalkylether und Polyoxyethylensorbitanalkylether bestehenden Gruppe auswählt.

15. Verfahren nach Anspruch 3, **dadurch gekennzeichnet, daß** man die Dispersion in der Stufe b) durch einen langsam rotierenden Rührer bewegt.

16. Verfahren nach einem der Ansprüche 1 bis 15, **dadurch gekennzeichnet, daß** man die Dispergierung in der Stufe b) bei einer Temperatur in dem Bereich von 60 bis 100 °C, vorzugsweise von 70 bis 85°C durchführt.

17. Verfahren nach einem der Ansprüche 1 bis 16, **dadurch gekennzeichnet, daß** man in der Stufe c1) auf eine Temperatur in dem Bereich von 0 bis 60 °C, vorzugsweise von 15 bis 50°C abkühlt.

18. Verfahren nach einem der Ansprüche 1 bis 17, **dadurch gekennzeichnet, daß** man die Verfestigung der Lösungsteilchen in der Dispersion der Stufe c2) bei einer Temperatur in dem Bereich von 30 bis 70 °C durchführt.

19. Verfahren nach einem der Ansprüche 1 bis 18, **dadurch gekennzeichnet, daß** man die Abtrennung in der Stufe c1) durch Filtrieren oder Zentrifugieren durchführt.

20. Verfahren nach einem der Ansprüche 1 bis 19, **dadurch gekennzeichnet, daß** man die Ausfällung in der Stufe c) mit wenigstens einem Fällungsmittels aus der aus Wasser, niederen Alkoholen und Polyolen mit einem Molekulargewicht < 600 bestehenden Gruppe durchführt.

21. Verfahren nach einem der Ansprüche 1 bis 20, **dadurch gekennzeichnet, daß** man als Lösungsmittel in der Stufe a) ein Einkomponentenlösungsmittel einsetzt, das man unter tertiären Aminoxiden, vorzugsweise N-Methylmorpholin-N-oxid-Monohydrat, oder Trifluoressigsäure auswählt.

22. Verfahren nach Anspruch 2, **dadurch gekennzeichnet, daß** man in der Stufe b) mit einem Volumenverhältnis von Celluloselösung zu Dispersionsmittel in dem Bereich von 1:1 bis 1:20, vorzugsweise 1:2 bis 1:5 arbeitet.

23. Verfahren nach einem der Ansprüche 1 bis 22, **dadurch gekennzeichnet, daß** man in der Stufe a) eine Lösung mit einer Cellulosekonzentration in dem Bereich von 1 bis 15 Masse-%, vorzugsweise von 2 bis 7 Masse-% bildet.

24. Verfahren nach einem der Ansprüche 1 bis 23, **dadurch gekennzeichnet, daß** man in der Stufe a) Cellulose mit einem Polymerisationsgrad von 200 bis 1.500 einsetzt.

25. Verfahren nach einem der Ansprüche 1 bis 24, **dadurch gekennzeichnet, daß** man in der Stufe b) ein Dispersionsmittel mit einer Viskosität in dem Bereich von 15 bis 5.10⁴ mPa.s einsetzt.

26. Verfahren nach einem der Ansprüche 1 bis 25, **dadurch gekennzeichnet, daß** man das in der Stufe c1) abgetrennte Dispersionsmittel oder das aus dem Gemisch der Stufe d) abgetrennte Dispersionsmittel in die Stufe b) zurückführt.

27. Verfahren nach einem der Ansprüche 1 bis 26, **dadurch gekennzeichnet, daß** man das in der Stufe d) anfallende Gemisch aus Lösungsmittel und Fällungsmittel trennt und das Lösungsmittel in die Stufe a) und das Fällungsmittel in die Stufe c) zurückführt.

28. Verfahren nach einem der Ansprüche 1 bis 27, **dadurch gekennzeichnet, daß** man die Abkühlung in der Stufe c1) unter Rührung durchführt.

## Claims

1. Process for producing regular porous pearl cellulose with a particle size in the range from 2 to 1,000 µm, **characterized in that**
a) a cellulose having a degree of polymerisation in the range from 150 to 2,000 is dissolved in a solvent to form a solution of 0.5 to 25 % by mass cellulose,
b) the cellulose solution is finely divided and dispersed in a dispersant which is immiscible with said solution and has a viscosity in the range from 10 to 80,000 mPa·s,
c) the dispersed solution particles are solidified to regular pearl particles by precipitating with a liquid precipitating agent miscible with the solvent
1) after cooling the dispersion to below the melting temperature of the cellulose solution and separating the frozen particles of the cellulose solution from the dispersant or
2) directly in the dispersion, and
d) the pearl particles are separated from the liquid mixture of solvent, precipitating agent and possibly dispersant.

2. Process according to claim 1 **characterized in that** for producing pearl cellulose with a particle size in the range from 2 to 50 µm the solution containing 0.5 to 15 % by mass cellulose is directly dispersed in the liquid inert medium and the dispersion is processed according to the steps c1) and d).

3. Process according to claim 1, **characterized in that** for producing pearl cellulose with a particle size in the range from 50 to 1,000 µm the cellulose solution in the step b)
is formed by pressure to at least one strand having a diameter in the range from 40 to 1,000 µm,
the solution strand is divided into defined sections by rotating cutting jets, and
the solution particles are caught in the dispersant and maintained in motion.

4. Process according to any of the claims 1 to 3 **characterized in that** the solvent is miscible with water, the dispersant is anhydrous and immiscible with water, and the precipitant consists at least partially of water.

5. Process according to any of the claims 1 to 4 **characterized in that** the solvent is salt-free and/or the precipitant is an aqueous salt solution.

6. Process according to any of the claims 1 to 5 **characterized in that** in the step a) 5 to 200 % by mass, referred to cellulose, of at least one inert solid are added.

7. Process according to any of the claims 1 to 6 **characterized in that** in the step a) a powdery material with particle diameters in the range from 50 to 3,000 nm is added.

8. Process according to claim 6 or 7 **characterized in that** the solid has particle diameters of < 40 nm and is selected from the group consisting of polysaccharides, such as starch, xanthane and galactomannanes, and inorganic compounds such as zinc oxide.

9. Process according to any of the claims 3 to 8 **characterized in that** in the step a) at least one inert diluent from the group consisting of dimethyl sulfoxide,lactames, polyethylen glycol and polypropylene glycol is added.

10. Process according to any of the claims 1 to 9 **characterized in that** the dispersant is selected from the group consisting of polyalkylsiloxanes, paraffins and polypropylene glycols.

11. Process according to any of the claims 2 to 8 **characterized in that** the step b) is carried out under action of a shearing field of a dispersing tool rotating at a speed n in the range from 10³ to 10⁴ min⁻¹.

12. Process according to claim 3 **characterized in that** the cellulose solution is extruded through at least one nozzle and the cutting jets are generated by a compressed inert liquid.

13. Process according to any of the claims 1 to 12 **characterized in that** in the step b) 1 to 30 % by mass referred to the cellulose, of at least one emulsifier are added.

14. Process according to claim 13 **characterized in that** the emulsifier is selected among nonionic surfactants of the group consisting of polyoxyalkylene alkylether, polyoxyethylene arylalkylether and polyoxyethylene sorbitanalkylether.

15. Process according to claim 3 **characterized in that** the dispersion in the step b) is moved by a slowly rotating agitator.

16. Process according to any of the claims 1 to 15 **characterized in that** the dispersion in the step b) is carried out at a temperature in the range from 60 to 100°C, preferably from 70 to 85°C.

17. Process according to any of the claims 1 to 16 **characterized in that** the cooling in the step c1) is carried out to a temperature in the range from 0 to 60°C, preferably from 15 to 50°C.

18. Process according to any of the claims 1 to 17 **characterized in that** the solidification of the solution particles in the dispersion of step c2) is carried out at a temperature in the range from 30 to 70°C.

19. Process according to any of the claims 1 to 18 **characterized in that** the separation in the step c1) is carried out by filtration or centrifugation.

20. Process according to any of the claims 1 to 19 **characterized in that** the precipitation in the step c) is carried out with at least one precipitant from the group consisting of water, lower alcohols and polyols with a molecular weight <600.

21. Process according to any of the claims 1 to 20 **characterized in that** as solvent in the step a) a one-component solvent is used which is selected among tertiary amine oxides, preferably N-methyl-morpholine-N-oxide monohydrate,oder trifluoroacetic acid.

22. Process according to claim 2 **characterized in that** the step b) is operated with a volume ratio of cellulose solution to dispersant in the range from 1:1 to 1:20, preferably 1:2 to 1:5.

23. Process according to any of the claims 1 to 22 **characterized in that** in the step a) a solution is formed with a cellulose content in the range from 1 to 15% by mass, preferably 2 to 7 % by mass.

24. Process according to any of the claims 1 to 23 **characterized in that** cellulose with a degree of polymerisation in the range from 200 to 1,500 is used in the step a).

25. Process according to any of the claims 1 to 24 **characterized in that** a dispersant having a viscosity in the range from 15 to 5 10⁴ mPa·s is used in the step b).

26. Process according to any of the claims 1 to 25 **characterized in that** the dispersant separated in the step c1) or from the mixture of step d) is recycled into the step b).

27. Process according to any of the claims 1 to 26 **characterized in that** the mixture of solvent and precipitant obtained in the step d) is separated and the solvent is recycled into step a) and the precipitant is recycled into step c).

28. Process according to any of the claims 1 to 27 **characterized in that** the cooling in the step c1) is carried out with agitation.

## Revendications

1. Procédé pour la fabrication de cellulose en perle poreuse et régulière présentant une plage de taille de particule de 2 à 1 000 µm, **caractérisé en ce que**
a) l'on dissout une cellulose présentant un degré de polymérisation s'inscrivant dans une plage de 150 à 2 000 dans un solvant en une solution à 0,5 à 25 % en masse,
b) l'on décompose la solution de cellulose en fines particules et disperse dans un milieu de dispersion présentant une viscosité s'inscrivant dans un domaine de 10 à 80 000 mPa.s qui n'est pas miscible avec celle-ci,
c) l'on compacte les particules de la solution dispersée
1) après refroidissement de la dispersion à la température de fusion de la solution de cellulose et séparation des particules de la solution de cellulose durcie par la solution de dispersion, ou que
2) directement dans la solution par précipitation avec une solution de précipitation liquide miscible avec le solvant pour obtenir des particules en forme de perle régulière, et que
d) l'on sépare les particules en forme de perle du mélange liquide à partir du solvant de la solution de précipitation, le cas échéant du milieu de dispersion.

2. Procédé selon la revendication 1, **caractérisé en ce que**, l'on disperse, pour la fabrication de cellulose en perle avec une plage de taille de particule allant de 2 à 50 µm, la solution avec 0,5 à 15 % en masse de cellulose directement dans un milieu liquide inerte et **en ce que** l'on retravaille la dispersion selon les étapes c1) et d).

3. Procédé selon la revendication 1, **caractérisé en ce que** l'on reforme, pour la fabrication de cellulose en perle présentant une plage de taille de particule de 50 à 1 000 µm, la solution de cellulose selon l'étape b)
par pression au niveau d'au moins un faisceau présentant un diamètre s'inscrivant dans une plage de 40 à 1 000 µm,
**en ce que** l'on décompose le faisceau de solution à l'aide d'un faisceau de coupe rotatif dans un segment défini, et
**en ce que** l'on recueille les particules de la solution dans le milieu de dispersion et que l'on les maintient en mouvement.

4. Procédé selon l'une quelconque des revendications 1 à 3, **caractérisé en ce que** le solvant est miscible avec de l'eau, **en ce que** la solution de dispersion ne contient pas d'eau et n'est pas miscible avec de l'eau et **en ce que** le milieu de précipitation se compose au moins en partie d'eau.

5. Procédé selon l'une quelconque des revendications 1 à 4, **caractérisé en ce que** le solvant ne contient pas de sel et/ou **en ce que** le milieu de précipitation est une solution saline aqueuse.

6. Procédé selon l'une quelconque des revendications 1 à 5, **caractérisé en ce que** l'on utilise dans l'étape a) 5 à 200 % en masse par rapport au poids de cellulose, au moins d'un solide inerte.

7. Procédé selon l'une quelconque des revendications 1 à 6, **caractérisé en ce que** l'on ajoute dans l'étape a) un matériau pulvérulent présentant des diamètres de particule s'inscrivant dans le domaine de 50 à 3 000 nm.

8. Procédé selon la revendication 6 ou 7, **caractérisé en ce que** l'on choisit le solide parmi les polysaccharides, comme l'amidon, le xanthane et le galactomannane et des composés inorganiques, comme l'oxyde de zinc, présentant des diamètres de particule de < 40 nm.

9. Procédé selon l'une quelconque des revendications 3 à 8, **caractérisé en ce que** l'on ajoute dans l'étape a) au moins un diluant inerte choisi dans le groupe se composant de sulfoxyde de diméthyle, de lactame, de polyéthylène glycol et de polypropylène glycol.

10. Procédé selon l'une quelconque des revendications 1 à 9, **caractérisé en ce que** l'on choisit le milieu de dispersion dans le groupe comprenant des polyalkylsiloxanes, des paraffines et des polypropylènes glycols.

11. Procédé selon l'une quelconque des revendications 2 à 8, **caractérisé en ce que** l'on réalise l'étape b) sous l'influence d'un champ de cisaillement d'un outil de dispersion qui tourne avec un nombre de tours n s'inscrivant dans une plage de 10³ à 10⁴ min⁻¹.

12. Procédé selon la revendication 3, **caractérisé en ce que** l'on extrude la solution de cellulose à l'aide d'au moins une buse et **en ce que** l'on produit le faisceau de coupe par un liquide inerte comprimé.

13. Procédé selon l'une quelconque des revendications 1 à 12, **caractérisé en ce que** l'on ajoute lors de l'étape b) 1 à 30 % en masse par rapport à la cellulose utilisée, au moins d'un émulsifiant.

14. Procédé selon la revendication 13, **caractérisé en ce que** l'émulsifiant est choisi parmi les détergents non-ioniques sélectionnés dans le groupe comprenant de l'éther alkylique de polyoxyéthylène, de l'éther arylalkylique de polyoxyéthylène et de l'éther alkylique de polyoxyéthylène sorbitane.

15. Procédé selon la revendication 3, **caractérisé en ce que** l'on agite la dispersion au cours de l'étape b) au moyen d'un agitateur à rotation lente.

16. Procédé selon l'une quelconque des revendications 1 à 15, **caractérisé en ce que** l'on réalise la dispersion dans l'étape b) à une température s'inscrivant dans un domaine de 60 à 100 °C, de préférence de 70 à 85 °C.

17. Procédé selon l'une quelconque des revendications 1 à 16, **caractérisé en ce que** le refroidissement dans l'étape c1) s'opère à une température s'inscrivant dans un domaine de 0 à 60 °C, de préférence de 15 à 50 °C.

18. Procédé selon l'une quelconque des revendications 1 à 17, **caractérisé en ce que** le compactage des particules de la solution est réalisé dans la dispersion de l'étape c2) à une température s'inscrivant dans un domaine de 30 à 70 °C.

19. Procédé selon l'une quelconque des revendications 1 à 18, **caractérisé en ce que** l'on réalise la séparation dans l'étape c1) par filtration ou centrifugation.

20. Procédé selon l'une quelconque des revendications 1 à 19, **caractérisé en ce que** l'on procède à la précipitation dans l'étape c) avec au moins une solution de précipitation choisie dans le groupe se composant d'eau, d'alcool inférieur et de polyol présentant un poids moléculaire de < 600.

21. Procédé selon l'une quelconque des revendications 1 à 20, **caractérisé en ce que** l'on utilise comme solvant dans l'étape a) un solvant unicomposant qui est choisi de préférence parmi les oxydes d'amine tertiaire, de préférence le monohydrate N-méthylmorpholine-N-oxyde, ou de l'acide trifluoroacétique.

22. Procédé selon la revendication 2, **caractérisé en ce que** l'on travaille dans l'étape b) avec un rapport en volume de la solution de cellulose sur le milieu de dispersion dans une plage de 1 : 1 à 1 : 20, de préférence de 1 : 2 à 1 : 5.

23. Procédé selon l'une quelconque des revendications 1 à 22, **caractérisé en ce que** l'on forme dans l'étape a) une solution présentant une concentration de cellulose s'inscrivant dans une plage de 1 à 15 % massiques, de préférence de 2 à 7 % en masse.

24. Procédé selon l'une quelconque des revendications 1 à 23, **caractérisé en ce que** l'on ajoute dans l'étape a) de la cellulose avec un degré de polymérisation de 200 à 1 500.

25. Procédé selon l'une quelconque des revendications 1 à 24, **caractérisé en ce que** l'on utilise dans l'étape b) un milieu de dispersion présentant une viscosité s'inscrivant dans un domaine de 15 à 5.10⁴ mPa.s.

26. Procédé selon l'une quelconque des revendications 1 à 25, **caractérisé en ce que** l'on réutilise le milieu de dispersion séparé dans l'étape c1) ou le milieu de dispersion séparé du mélange de l'étape d) dans l'étape b).

27. Procédé selon l'une quelconque des revendications 1 à 26, **caractérisé en ce que** l'on sépare le mélange précipité dans l'étape d) du solvant ou de la solution de précipitation et ce que l'on réutilise le solvant dans l'étape a) et la solution de précipitation dans l'étape c).

28. Procédé selon l'une quelconque des revendications 1 à 27, **caractérisé en ce que** l'on réalise le refroidissement dans l'étape c1) sous agitation.
